# EUROPEAN PATENT APPLICATION

(11) **EP 4 241 654 A1**
(43) Date of publication of application: **13.09.2023**
(21) Application number: 21889593.6
(22) Date of filing: 04.11.2021
(51) Int. Cl.: A61B 5/00, A61B 5/145, A61B 5/1455

(54) **PHOTOACOUSTIC DIAGNOSTIC DEVICE AND METHOD**

(30) Priority: 06.11.2020 KR 20200147505
(71) Applicant: HME Square Co., Ltd., Daejeon 34138 (KR)
(72) Inventor: KHANG, Yoonho, Yongin-si Gyeonggi-do 16822 (KR)
(74) Representative: dompatent von Kreisler Selting Werner - Partnerschaft von Patent- und Rechtsanwälten mbB
(86) International application number: PCT/KR2021/015931
(87) International publication number: WO 2022/098128

(57) **Abstract**

The present invention relates to a photoacoustic diagnosis apparatus and method for measuring blood sugar in blood by measuring a change in shape of a membrane by sound waves, and more particularly, to a photoacoustic diagnosis apparatus and method for adjusting a frequency response of a diagnosis apparatus by adjusting a size of a membrane.

A photoacoustic diagnosis apparatus according to an embodiment of the present invention includes a light source radiating sound by radiating light to a subject, a plurality of sound receivers provided with a membrane whose shape is changed by the sound, and a measuring unit measuring a change in shape of the membrane, in which the membranes of the plurality of sound receivers have different sizes.

## Description

### [Technical Field]

The present invention relates to a photoacoustic diagnosis apparatus and method for measuring blood sugar in blood by measuring a change in shape of a membrane by sound waves, and more particularly, to a photoacoustic diagnosis apparatus and method for adjusting a frequency response of a diagnosis apparatus by adjusting a size of a membrane.

### [Background Art]

A photoacoustic diagnosis technology is a technology for non-invasively characterizing living tissue using a photoacoustic effect. For the photoacoustic diagnosis, when a short electromagnetic pulse from a laser is incident on living tissue, a portion of energy is absorbed by a tissue and then converted into heat, causing an instantaneous thermoelastic expansion. As a result, ultrasonic waves having a wide band of frequencies may be emitted, and detected by ultrasonic transducers in various directions and converted into images.

The photoacoustic diagnosis technology has the advantage of being able to combine the characteristics of optical imaging and ultrasound imaging because it converts electromagnetic waves into ultrasonic waves and detects these ultrasonic waves. A contrast ratio of pure optical imaging techniques is very high compared to ultrasound imaging, but has the disadvantage of being able to image only up to a certain depth from a surface of a living body due to high light scattering in soft tissue. In contrast, the ultrasound imaging has a spatial resolution high enough to be used for fetal examination. The photoacoustic imaging may overcome the low imaging depth, which is a disadvantage of the optical imaging, by ultrasonic conversion by the photoacoustic effect, thereby realizing the high optical contrast and high spatial resolution at the same time.

The apparatus that implements the photoacoustic diagnosis technology largely includes an optical unit that irradiates laser and a transducer that measures ultrasonic waves. Transducers for measuring ultrasonic waves include a piezo method and a micro electro mechanical systems (MEMS) method. The piezoelectric method measures ultrasonic waves by forming a potential in a piezoelectric material by a pressure caused by ultrasonic waves and measuring a voltage which is a potential difference. The MEMS method measures ultrasonic waves by changing a shape of a membrane film by the pressure caused by the ultrasonic waves and measuring a change in capacitance due to the change in the shape.

Since the above-described transducer includes an optical unit and a separate module, the photoacoustic diagnosis apparatus having the above-described transducer has problems in that it is difficult to reduce the size and the convenience of use is low.

### [Disclosure]

### [Technical Problem]

In order to solve the above-described problems, an object of the present invention is to provide a photoacoustic diagnosis apparatus and method for matching a photoacoustic peak of blood sugar by adjusting a resonance frequency of a transducer through membrane size control.

### [Technical Solution]

In an embodiment of the present invention, a photoacoustic diagnosis apparatus is provided.

According to an embodiment of the present invention, a photoacoustic diagnosis apparatus includes a light source radiating sound by radiating light to a subject, a plurality of sound receivers provided with a membrane whose shape is changed by the sound, and a measuring unit measuring a change in shape of the membrane, in which the membranes of the plurality of sound receivers may have different sizes.

In the photoacoustic diagnosis apparatus according to the embodiment of the present invention, each resonance frequency of the sound receiver may be matched to a photoacoustic peak of blood sugar.

In an embodiment of the present invention, a photoacoustic diagnosis method is provided.

According to an embodiment of the present invention, a photoacoustic diagnosis method may include radiating light to a subject using a light source, collecting sound emitted from the subject through a plurality of sound receivers provided with a membrane, measuring a change in shape of the membrane, and analyzing a presence and amount of blood sugar contained in a blood by analyzing the change in the shape of the membrane.

According to an embodiment of the present invention, the photoacoustic diagnosis method may further include matching each resonance frequency of the sound receiver to a photoacoustic peak of blood sugar by adjusting a size of the membrane of the sound receiver.

In an embodiment of the present invention, there may be provided a computer-readable recording medium on which a program for realizing the method described above is recorded.

### [Advantageous Effects]

According to a photoacoustic diagnosis apparatus according to the present invention, it is possible to improve a frequency response of the photoacoustic diagnosis apparatus by adjusting a resonance frequency of a transducer by adjusting a size of a membrane.

Effects which can be achieved by the present disclosure are not limited to the above-described effects. That is, other objects that are not described may be obviously understood by those skilled in the art to which the present disclosure pertains from the following description.

### [Brief Description of Drawings]

FIG. 1 is an overall configuration diagram of a conventional photoacoustic diagnosis apparatus.
FIG. 2 is a block diagram of a photoacoustic diagnosis apparatus according to an embodiment of the present invention.
FIG. 3 is a diagram illustrating an embodiment of a sound receiver of the photoacoustic diagnosis apparatus according to the present invention.
FIG. 4 is a diagram illustrating a frequency response of the sound receiver of the photoacoustic diagnosis apparatus according to the embodiment of the present invention.
FIG. 5 is a graph illustrating an example of a photoacoustic signal caused by blood sugar.
FIG. 6 is a flowchart of a photoacoustic diagnosis method according to an embodiment of the present invention.

### [Best Mode]

In an embodiment of the present invention, a photoacoustic diagnosis apparatus is provided.

According to an embodiment of the present invention, a photoacoustic diagnosis apparatus includes a light source radiating sound by radiating light to a subject, a plurality of sound receivers provided with a membrane whose shape is changed by the sound, and a measuring unit measuring a change in shape of the membrane, in which the membranes of the plurality of sound receivers may have different sizes.

In the photoacoustic diagnosis apparatus according to the embodiment of the present invention, each resonance frequency of the sound receiver may be matched to a photoacoustic peak of blood sugar.

In an embodiment of the present invention, a photoacoustic diagnosis method is provided.

According to an embodiment of the present invention, a photoacoustic diagnosis method may include radiating light to a subject using a light source, collecting sound emitted from the subject through a plurality of sound receivers provided with a membrane, measuring a change in shape of the membrane, and analyzing a presence and amount of blood sugar contained in a blood by analyzing the change in the shape of the membrane.

According to an embodiment of the present invention, the photoacoustic diagnosis method may further include matching each resonance frequency of the sound receiver to a photoacoustic peak of blood sugar by adjusting a size of the membrane of the sound receiver.

In an embodiment of the present invention, there may be provided a computer-readable recording medium on which a program for realizing the method described above is recorded.

### [Most for Invention]

Hereinafter, embodiments of the present disclosure will be described in detail with reference to the accompanying drawings so that those skilled in the art to which the present disclosure pertains may easily practice the present invention. However, the present disclosure may be implemented in various different forms and is not limited to exemplary embodiments described herein. In addition, in the drawings, portions unrelated to the description will be omitted to clearly describe the present disclosure, and similar portions will be denoted by similar reference numerals throughout the specification.

After terms used in the present specification are briefly described, the present disclosure will be described in detail.

General terms that are currently widely used are selected as terms used in the present invention in consideration of functions in the present invention but may be changed depending on the intention of those skilled in the art or a judicial precedent, the emergence of a new technique, and the like. In addition, in a specific case, terms arbitrarily chosen by an applicant may exist. In this case, the meaning of such terms will be mentioned in detail in a corresponding description portion of the present disclosure. Therefore, the terms used in exemplary embodiments of the present disclosure should be defined on the basis of the meaning of the terms and the contents throughout the present disclosure rather than simple names of the terms.

Throughout the specification, unless otherwise specified, "including" any component means that other components may be further included rather than excluding other components. In addition, terms "-er/or," "module," and the like, described in the specification refer to a processing unit of at least one function or operation and may be implemented by hardware or software or a combination of hardware and software. In addition, throughout the present specification, when any one part is referred to as being "connected to" another part, it means that any one part and another part are "directly connected to" each other or are "connected to" each other with the other part interposed therebetween.

Hereinafter, the present disclosure will be described in detail with reference to the accompanying drawings.

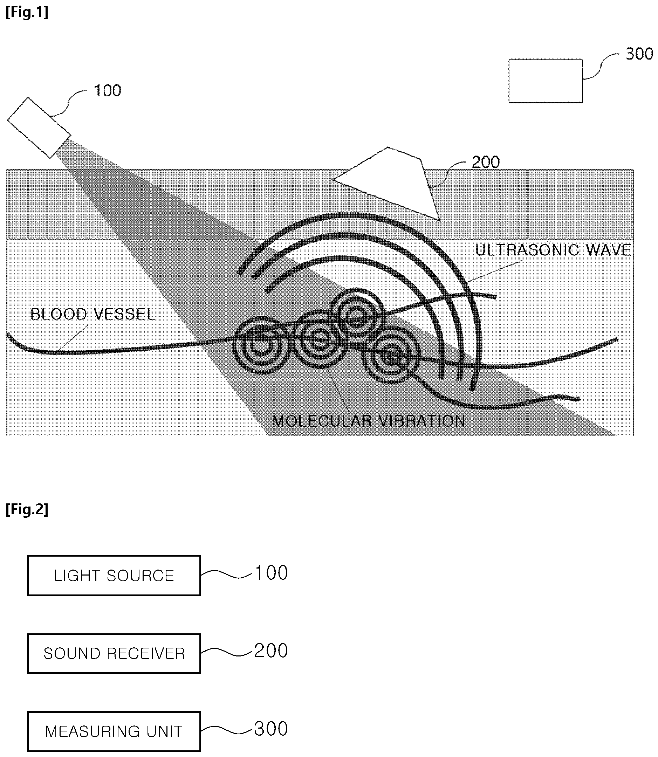

FIG. 1 is an overall configuration diagram of a photoacoustic diagnosis apparatus according to an embodiment of the present invention, and FIG. 2 is a block diagram of a photoacoustic diagnosis apparatus according to an embodiment of the present invention.

Referring to FIGS. 1 and 2, the photoacoustic diagnosis apparatus according to the embodiment of the present invention may include a light source 100 radiating sound by radiating light to a subject, a plurality of sound receivers 200 provided with a membrane whose shape is changed by the sound, and a measuring unit 300 measuring a change in shape of the membrane, in which the membranes of the plurality of sound receivers 200 may have different sizes.

The light source 100 may output laser light, and the laser light may be infrared ray.

The laser output from the light source 100 is irradiated to the subject to vibrate molecules included in the subject, and ultrasonic waves are emitted by the vibration of the molecules.

The photoacoustic diagnosis apparatus may include a plurality of sound receivers 200, and a resonance frequency (RF) of each sound receiver 200 may be variously set by forming different sizes of membranes included in each sound receiver 200.

That is, the photoacoustic diagnosis apparatus according to the present invention may have a plurality of resonance frequencies by including a plurality of sound receivers 200 having different sizes, and measure a photoacoustic signal caused by blood glucose using the resonance frequencies.

The measuring unit 300 may use an optical method of measuring the change in the shape of the membrane by measuring capacitance, and measuring and analyzing light reflected by irradiating the light to the sound receiver 200 to measure the change in the shape of the membrane.

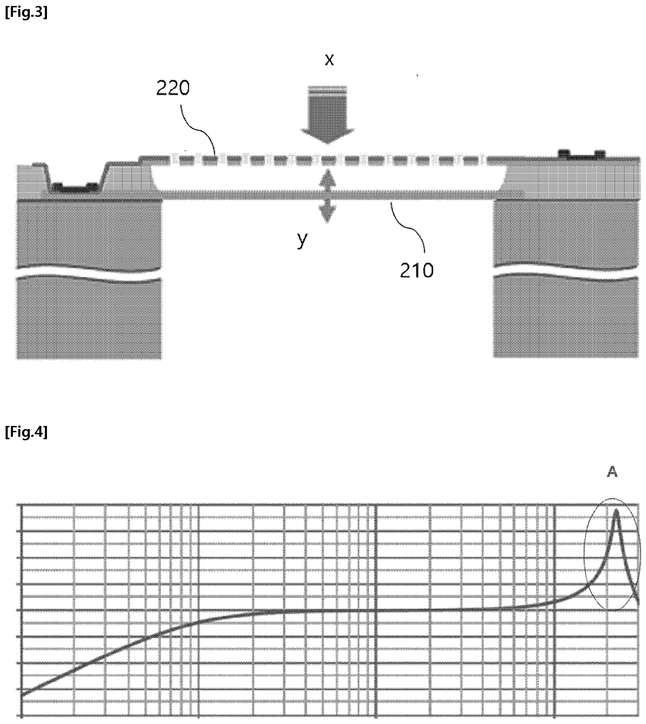

FIG. 3 illustrates an embodiment of the sound receiver in the photoacoustic diagnosis apparatus according to the present invention.

Referring to FIG. 3, the sound receiver 200 may further include a membrane for detecting sound and a protection unit for protecting the membrane. In the photoacoustic diagnosis apparatus according to the present invention, the sound may correspond to ultrasonic waves.

The membrane may vibrate in a vertical direction Y according to an applied sound X. A vertical vibration width of the membrane may vary depending on intensity of the sound X. For example, as the intensity of the sound X increases, the membrane will vibrate up and down with a larger width.

The membrane may serve to detect the intensity of the sound X applied from the outside, and may be configured as a thin membrane so as to vibrate with as large an amplitude as possible for the sound X applied from the outside.

The membrane may be formed of single-crystalline silicon, polycrystalline silicon, silicon nitride, silicon oxide, aluminum and other metal thin film materials mainly used in the MEMS process, carbon or compounds containing carbon, and a stacked form of the above materials.

The protection unit may preferably be formed of a material having a structure that protects the membrane from external impact and does not hinder the transmission of sound to the membrane. For example, a mesh material having a net structure may be used.

FIG. 4 is a diagram illustrating a frequency response of the sound receiver of the photoacoustic diagnosis apparatus according to the embodiment of the present invention.

A frequency response of one sound receiver 200 may be seen in FIG. 4. In FIG. 4, a horizontal axis is a frequency (Hz), and a vertical axis is amplitude (dB). The resonance frequency means a frequency having significantly larger amplitude than other frequencies, and in FIG. 4, A corresponds to the resonance frequency.

The sound receiver 200 may include one membrane, and one membrane has one resonance frequency. For example, when the photoacoustic diagnosis apparatus includes six sound receivers 200, the photoacoustic diagnosis apparatus may include six membranes and will have six resonance frequencies.

The photoacoustic diagnosis apparatus according to the embodiment of the present invention may include six sound receivers 200, and each resonance frequency of the sound receiver 200 may be matched to a photoacoustic peak of blood sugar.

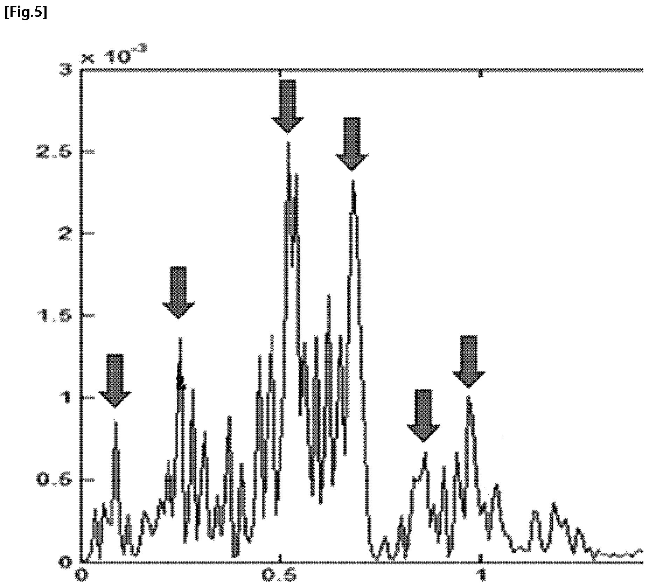

FIG. 5 is a graph illustrating an example of a photoacoustic signal caused by blood sugar.

In FIG. 5, the horizontal axis is the frequency (HZ), and the vertical axis is a power spectral density (W/Hz).

Referring to FIG. 5, the photoacoustic signal may have six peaks, and the forms of the frequency and shape of the peaks may vary depending on measurement conditions.

That is, the resonance frequency of the sound receiver may be matched by selecting the desired number of peaks among the main peaks detected when a laser is irradiated to blood. It can be seen that the greater the amplitude of the peak measured at the matched resonance frequency, the greater the amount of blood sugar.

The photoacoustic diagnosis apparatus according to the present invention includes the plurality of sound receivers 200, and may match the resonance frequencies of each sound receiver 200 to the peak frequency of the blood sugar, so the sound receiver exhibiting large amplitude may measure even a small signal caused by low-concentration blood sugar or blood sugar in a blood vessel far from a skin, thereby precisely measuring the amount of blood sugar in the blood.

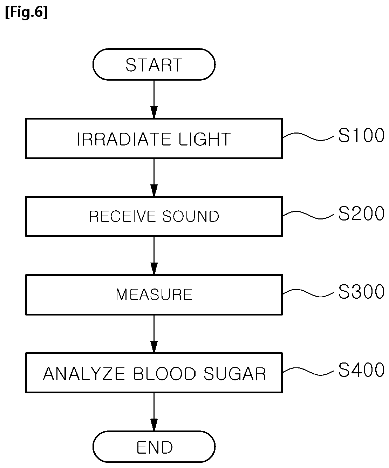

FIG. 6 is a flowchart of a photoacoustic diagnosis method according to an embodiment of the present invention.

Referring to FIG. 6, the photoacoustic diagnosis method for measuring blood sugar using a photoacoustic diagnosis apparatus according to the embodiment of the present invention may include radiating light to a subject using a light source 100 (S100), collecting sound emitted from the subject through a plurality of sound receivers 200 provided with a membrane (S200), measuring a change in shape of the membrane (S300), and analyzing a presence and amount of blood sugar contained in a blood by analyzing the change in the shape of the membrane (S400).

According to an embodiment of the present invention, the photoacoustic diagnosis method may further include matching each resonance frequency of the sound receiver 200 to the photoacoustic peak of blood sugar by adjusting the size of the membrane of the sound receiver 200 (S110).

Regarding the method according to an embodiment of the present invention, the contents of the above-described apparatus may be applied. Therefore, the description of the same contents as those of the above-described apparatus in relation to the method will be omitted.

The above description of the present invention is for illustrative purposes, and those skilled in the art to which the present invention pertains will understand that it may be easily modified to other specific forms without changing the technical spirit or essential features of the present invention. Therefore, it should be understood that the above-mentioned embodiments are exemplary in all aspects but are not limited thereto. For example, each component described as a single type may be implemented in a distributed manner, and similarly, components described as distributed may be implemented in a combined form.

It is to be understood that the scope of the present invention will be defined by the claims rather than the above-described description and all modifications and alternations derived from the claims and their equivalents are included in the scope of the present invention.

## Claims

1. A photoacoustic diagnosis apparatus, comprising:
a light source radiating sound by radiating light to a subject;
a plurality of sound receivers provided with a membrane whose shape is changed by the sound; and
a measuring unit measuring a change in shape of the membrane,
wherein the membranes of the plurality of sound receivers have different sizes.

2. The photoacoustic diagnosis apparatus of claim 1, wherein each resonance frequency of the sound receiver is matched to a photoacoustic peak of blood sugar.

3. A photoacoustic diagnosis method for measuring blood sugar using a photoacoustic diagnosis apparatus, the photoacoustic diagnosis method comprising:
radiating light to a subject using a light source;
collecting sound emitted from the subject through a plurality of sound receivers provided with a membrane;
measuring a change in shape of the membrane; and
analyzing a presence and amount of blood sugar contained in a blood by analyzing the change in the shape of the membrane.

4. The photoacoustic diagnosis method of claim 3, further comprising:
matching each resonance frequency of the sound receiver to a photoacoustic peak of blood sugar by adjusting a size of the membrane of the sound receiver.

5. A computer-readable recording medium on which a program for realizing the method according to any one of claims 3 to 4 is recorded.
